# EUROPEAN PATENT SPECIFICATION

(11) **EP 3 512 869 B1**
(45) Date of publication and mention of the grant of the patent: **28.02.2024**
(21) Application number: 17768767.0
(22) Date of filing: 15.09.2017
(51) Int. Cl.: C07K 14/00

(54) **MAGNETIC PARTICLE-BINDING PEPTIDES**
MAGNETISCHE PARTIKELBINDUNGSPEPTIDE
PEPTIDES DE LIAISON MAGNÉTIQUE DE PARTICULES

(30) Priority: 16.09.2016 EP 16189240; 21.09.2016 EP 16189914
(43) Date of publication of application: 24.07.2019
(73) Proprietor: Technische Universität München, 80333 München (DE)
(72) Inventor: BERENSMEIER, Sonja, 81829 München (DE); BLANK-SHIM, Silvia, 63743 Aschaffenburg (DE); SCHWAMINGER, Sebastian, 8010 Graz (AT); FRAGA GARCIA, Paula, 85354 Freising (DE); WENZEL, Wolfgang, 76646 Bruchsal (DE); FINK, Karin, 76646 Bruchsal (DE); ANAND, Priya, 67122 Altrip (DE); BORKOWSKA-PANEK, Monika, 69190 Walldorf (DE)
(74) Representative: Vossius & Partner Patentanwälte Rechtsanwälte mbB
(86) International application number: PCT/EP2017/073291
(87) International publication number: WO 2018/050826

(56) References cited:
- WO-A1-2007/133153
- WO-A1-2013/024047
- US-A- 5 968 753
- US-A1- 2008 241 964
- BARCH MARIYA ET AL: "Screen-Based Analysis of Magnetic Nanoparticle Libraries Formed Using Peptidic Iron Oxide Ligands", September 2014 (2014-09), JOURNAL OF THE AMERICAN CHEMICAL SOCIETY, VOL. 136, NR. 36, PAGE(S) 12516-12519, XP002766578, ISSN: 0002-7863 figure 1
- COLOMBO MIRIAM ET AL: "Biological applications of magnetic nanoparticles", 2012, CHEMICAL SOCIETY REVIEWS, VOL. 41, NR. 11, PAGE(S) 4306-4334, XP002766579, ISSN: 0306-0012(print) page 4315, left-hand column, paragraph 2 - page 4316, left-hand column, paragraph 1
- GARRIS, JOHN P.; SIKES, C. S.: "Use of polyamino acid analogs of biomineral proteins in dispersion of inorganic particulates important to water treatment", COLLOIDS AND SURFACES, A: PHYSICOCHEMICAL AND ENGINEERING ASPECTS, vol. 80, 1 January 1993 (1993-01-01), pages 103-112, XP002774853,
- ANDREW K JOHNSON ET AL: "Novel method for immobilization of enzymes to magnetic nanoparticles", JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, vol. 10, no. 6, 7 December 2007 (2007-12-07), pages 1009-1025, XP019612169, ISSN: 1572-896X

## Description

The present disclosure relates to a peptide consisting of a sequence of 5 to 30, preferably 6 to 12, most preferably 10 to 12 amino acids, wherein (a) at least 2/3 of said amino acids have a functional group or side chain which is negatively charged at neutral pH; (b) amino acids which do not have a functional group or side chain which is negatively charged at neutral pH, if present, meet one or both of requirements (i) and (ii): (i) none of them has a functional group or side chain which is positively charged at neutral pH; and (ii) at least one of them has a side chain which does not bear a net charge at neutral pH or which has a functional group or side chain that does not bear a net charge at neutral pH.

The present invention is defined by the claims.

Accordingly, the present invention relates to a peptide consisting of a sequence of 5 to 30, preferably 6 to 12, most preferably 10 to 12 amino acids, wherein said peptide is capable of binding to a surface comprising magnetic iron oxide, and wherein (a) at least 2/3 of said amino acids are selected from Asp and Glu; and (b) at least one amino acid which has a side chain which does not bear a net charge at neutral pH is present, selected from Gly, Ser, Ala, Asn, Cvs, Gln, His, Ile, Leu, Met, Phe, Pro, Thr, Trp, Tyr, Val and selenocystein; and flanked on either side by at least one, preferably at least two amino acids as defined in (a).

Magnetic nanoparticles (MNPs) are widely used for purification of proteins, nucleic acids and other biological molecules (Fraga Garcia, P., et al. (2015): High-gradient magnetic separation for technical scale protein recovery using low cost magnetic nanoparticles, Separation and Purification Technology 150, pp. 29-36; Rittich, B. et al. (2013): SPE and purification of DNA using magnetic particles, Journal of Separation Science 36 (15), pp. 2472-2485; Colombo, M., et al. (2012): Biological applications of magnetic nanoparticles, Chem. Soc. Rev. 41 (11), pp. 4306-4334). MNP are also useful for biomedical applications such as optical imaging (Le Sage, D., et al. (2013): Optical magnetic imaging of living cells, Nature 496 (7446), pp. 486-489), drug delivery and hyperthermia and, as well as contrast agents for magnetic resonance imaging (Boyer, C., et al. (2010): The design and utility of polymer-stabilized iron-oxide nanoparticles for nanomedicine applications, NPG Asia Mater 2, pp. 23-30; Shinkai, M. (2002): Functional magnetic particles for medical application, Journal of Bioscience and Bioengineering 94 (6), pp. 606-613; Senpan, A., et al. (2009): Conquering the Dark Side: Colloidal Iron Oxide Nanoparticles, ACS Nano 3 (12), pp. 3917-3926)). Small magnetite particles naturally occur in magnetotactic bacteria, helping the hosts to orient themselves in the geomagnetic field (Mann, S., et al. (1984): Structure, Morphology and Crystal-Growth of Bacterial Magnetite, Nature 310 (5976), pp. 405-407).

MNP can be manufactured in sizes ranging from a few to hundreds of nanometers, which results in a large specific surface area. MNPs show superparamagnetic behavior, which allows their manipulation by an external magnetic field (Pankhurst, Q. A., et al. (2003): Applications of magnetic nanoparticles in biomedicine, Journal of Physics D: Applied Physics 36 (13), pp. R167; Zhao, F. Y., et al. (2012): Preparation and magnetic properties of magnetite nanoparticles, Mater Lett 68, pp. 112-114).

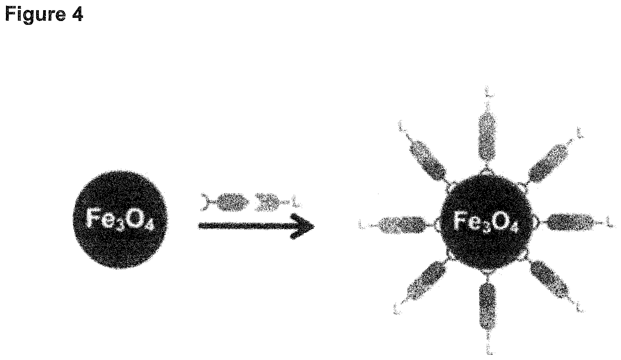

In order to be used in a number of applications, MNPs must be functionalized to selectively bind to their respective interaction partners. This can presently be achieved by attaching metal ion chelating molecules like nitrilotriacetic acid to the MNP surface so that His-tagged biomolecules can be bound (Block, H. et al. 2009: Chapter 27, Immobilized-Metal Affinity Chromatography (IMAC); Richard R. Burgess, Murray P. Deutscher (Eds.): Guide to protein purification, vol. 463. 2nd ed. Amsterdam, Boston: Elsevier/Academic Press (Methods in Enzymology, v. 463), pp. 439-473). Drawbacks of this method are the leakage of toxic metal ions and instability of the surface functionalization (Gaberc-Porekar, V. and Menart, V. 2005: Potential for Using Histidine Tags in Purification of Proteins at Large Scale; Chem. Eng. Technol. 28 (11), pp. 1306-1314); Hearn, M. and Acosta, D. 2001: Applications of novel affinity cassette methods: use of peptide fusion handles for the purification of recombinant proteins; in J. Mol. Recognit. 14 (6), pp. 323-369). Other surface modifications for protein adsorption on magnetic particles use glutathione, streptavidin, biotin or protein A, but all of these lead to high costs of over 400 to several thousand euros per gram (Franzreb, M. et al. 2006: Protein purification using magnetic adsorbent particles; in Appl Microbiol Biot 70 (5), pp. 505-516). This principle is illustrated in Figure 4. Problems of covalent immobilization techniques such as amino-silanization are the high complexity (Uzun, K. et al. 2010: Covalent immobilization of invertase on PAMAM-dendrimer modified superparamagnetic iron oxide nanoparticles; in Journal of Nanoparticle Research 12 (8), pp. 3057-3067) and loss of enzymatic activity due to the reaction of catalytic amino acids (Johnson, A. et al. 2008: Novel method for immobilization of enzymes to magnetic nanoparticles; in J Nanopart Res 10 (6), pp. 1009-1025).

For these reasons it would be advantageous to overcome the requirement of application-specific functionalization and to engineer tags such as peptide sequences that can bind to MNPs and, in case of the tags being peptides, can directly be encoded by those nucleic acids which also encode the polypeptide of interest for a given application.

In view of the deficiencies of the prior art, the technical problem underlying the present invention can be seen in the provision of improved means and methods of binding molecules such as biomolecules or analytes in general to iron oxide-containing surfaces, especially magnetic particles. This technical problem is solved by the subject-matter of the attached claims.

Accordingly, in a first aspect, the present invention disclosure relates to a peptide consisting of a sequence of 5 to 30, preferably 6 to 12, most preferably 10 to 12 amino acids, wherein (a) at least 2/3 of said amino acids have a functional group or side chain which is negatively charged at neutral pH; (b) amino acids which do not have a functional group or side chain which is negatively charged at neutral pH, if present, meet one or both of requirements (i) and (ii): (i) none of them has a functional group or side chain which is positively charged at neutral pH; and (ii) at least one of them has a side chain which does not bear a net charge at neutral pH or does not bear a net charge at neutral pH.

Described herein is also a peptide consisting of a sequence of 5 to 30, preferably 6 to 12, most preferably 10 to 12 amino acids, wherein (a) at least 2/3 of said amino acids have a functional group or side chain which is negatively charged at neutral pH; (b) amino acids which do not have a functional group or side chain which is negatively charged at neutral pH, if present, meet one or both of requirements (i) and (ii): (i) none of them has a functional group or side chain which is positively charged at neutral pH; and (ii) at least one of them has a side chain which does not bear a net charge at neutral pH or which has a functional group or side chain that does not bear a net charge at neutral pH.

The term "peptide" has its art-established meaning. It relates to a polycondensate of amino acids, the number of amino acids being 30 or less. In accordance with the disclosure, peptides have length between 5 and 30 amino acids. Envisaged lengths are 5, 6, 7, 8, 9, 10, 11, 12, 13, 14, 15, 16, 17, 18, 19, 20, 21, 22, 23, 24, 25, 26, 27, 28, 29 and 30 amino acids. Peptides may be chemically synthesized or translated from nucleic acids encoding them.

The term "peptide" as used herein does not imply a limitation to α-amino acids. Therefore, the charge status of the amino acids is a feature of a functional group comprised in a given amino acid. To the extent the amino acids are α-amino acids, the term "side chain" has its usual meaning and designates group R in the following generic formula: -NH-CHR-CO-. The side chain may carry a functional group which is charged at neutral pH.

Amino acids are preferably α-amino acids, wherein the 20 proteinogenic amino acids are especially preferred. To the extent use is made of proteinogenic amino acids, peptides of the present disclosure as well as fusion constructs, namely polypeptides and proteins as disclosed further below can be encoded by nucleic acids. Having said that, it is also envisaged to use other amino acids, wherein said other amino acids include α-amino acids which do not belong to the set of 20 proteinogenic amino acids such as pyrrolysine and selenocysteine. Among α-amino acids, preference is given to L-amino acids. At the same time, the use of D-amino acids is also envisaged. Moreover, one or more amino acids which are not α-amino acids, such as β-amino acids may be present. Preference is given to absence of non-proteinogenic amino acids. Yet, any of the above-mentioned non-proteinogenic amino acids may be present, preferably in small numbers such as 1, 2, 3, 4 or 5. It is not excluded that also 6, 7, 8, 9 or 10 non-proteinogenic amino acids are comprised in peptides in accordance with the first aspect Said peptides may entirely consist of non-proteinogenic amino acids.

In accordance with the first aspect, at least 2/3 of the amino acids are required to be amino acids with a functional group or side chain that is negatively charged at neutral pH. It is understood that at least in a preferred case said amino acids do not contain a functional group or side chain which is positively charged at neutral pH.

Among the proteinogenic amino acids, this requirement is fulfilled by Asp and Glu. If a peptide consists of 9 amino acids, this means that, in accordance with requirement (a), 6, 7, 8 or all 9 amino acids have a side chain which is negatively charged at neutral pH.

To the extent amino acids are comprised in said peptides which do not fulfill requirement (a), item (b) of the first aspect imposes restrictions on these further amino acids. It follows from conditions (i) and (ii) that up to 1/3 neutral or polar amino acids are possible. If both requirements (i) and (ii) are fulfilled, no amino acids are present which are positively charged at neutral pH. To the extent one or more amino acids with a side chain that is positively charged at neutral pH are present, this entails the requirement for a presence of at least one amino acid which does not bear a net charge at neutral pH or which has a functional group or side chain that does not bear a net charge at neutral pH. If the sequence of a peptide in accordance with the first aspect does not contain any amino acid with a side chain that is positively charged at neutral pH, there are no further limitations on its composition other than the limitation of (a).

It is understood that at least in a preferred embodiment case said amino acids which have a functional group or side chain that is positively charged at neutral pH do not contain a functional group or side chain which is negatively charged at neutral pH.

Preferred amino acids which are positively charged at neutral pH are Lys, Arg and His.

Whether or not a given amino acid meets any one of the above criteria, may determined by assessing an aqueous solution of the amino acid at issue in its monomeric form, i.e., with free amino and carboxy terminus. Alternatively, N-terminus and C-terminus may be protected, for example, the N-terminus may be acetylated and the C-terminus may amidated. p*K*ₐ value(s) of an amino acid can be determined by acid/base titration. The p*K*ₐ value in turn determines the charge status at a given pH value.

Preferred specific amino acids meeting the above requirements are the subject of preferred cases which are disclosed further below.

Related to the first aspect, the disclosure also relates to, in a second aspect, a peptide consisting of a sequence of 5 to 30, preferably 6 to 12, most preferably 10 to 12 amino acids, wherein said sequence consists of at least one (S) region and, optionally, at least one (W) region, wherein, if at least one (W) region is present, the (S) and (W) regions are alternating; with (S) representing a region consisting of negatively charged and/or positively charged amino acids, and (W) representing a region consisting of neutral, hydrophobic and/or polar amino acids, wherein (a) each (S) region independently consists of more than 2 negatively charged and/or more than 2 positively charged amino acids, (W) regions independently consist of 1, 2 or 3 amino acids; and the total number of positively charged and negatively charged amino acids in said sequence is greater than 5; or (b) each (S) region independently consists of more than 2 negatively charged and/or 0, 1 or 2 positively charged amino acids; (W) regions independently consist of 1, 2 or 3 amino acids; the total number of positively charged amino acids in said sequence is 0, 1 or 2; the total number of negatively charged amino acids in said sequence is at least 5; and said peptide has an isoelectric point (pl) of less than about 5.0.

This aspect of the disclosure introduces the notion of regions within the sequence of the peptide. In particular, the designations (S) and (W) refer to groups of adjacent amino acids within said peptide which confer strong binding to a surface comprising iron oxide and groups which consist of adjacent amino acids which confer weak binding to such surface, respectively.

Designations of amino acids relating to the charge status, especially the designations "negatively charged", "positively charged" and "neutral" refer to the charge status at neutral pH. The same applies to polar amino acids.

The distinction between items (a) and (b) is *inter alia* based on the number of allowed amino acids bearing a positive charge. Item (a) allows for more than two positively charged amino acids per (S) region, whereas item (b) confines the number of positively charged amino acids per (S) region to 2. This will be discussed in more detail further below. Higher numbers of positively charged amino acids in accordance with item (a) confer particularly strong binding to surfaces comprising iron oxide. Such particularly strong binding can be viewed, for practical purposes, as being irreversible. This is also illustrated in the Examples enclosed herewith.

On the other hand, by confining the number of positively charged amino acids to lower values (as in item (b)) provides for reversible binding to the mentioned surfaces. Also the notion of reversibility will be discussed in more detail below.

Examples of alternating (S) and (W) regions include (S)(W), (W)(S), (S)(W)(S), (W)(S)(W), (S)(W)(S)(W) and (W)(S)(W)(S). A preferred pattern is (S)(W)(S). This applies to all aspects of the disclosure.

In a preferred case of the peptide in accordance with the first aspect, homopolymers of amino acids which have a functional group or side chain which is negatively charged at neutral pH are excluded. Particularly preferred is that homopolymers of Asp and/or of Glu are excluded. Especially preferred is that the homodecamer DDDDDDDDDD is excluded.

In a further preferred case, the fraction of amino acids which have a functional group or side chain which is positively charged at neutral pH is (a) in the range between zero and 0.2, preferably zero; or (b) greater than 0.2.

This preferred case is related to the distinction between items (a) and (b) of the second aspect. In particular, a low or zero fraction of amino acids which have a side chain which is positively charged at neutral pH defines reversible binders. Item (b) on the other hand defines irreversible binders. In either case, binding is preferably non-covalent.

An example of a peptide sequence which has a fraction of 0.2 in accordance with item (a) is a sequence consisting of 10 amino acids, 2 of which have a side chain which is positively charged at neutral pH.

In a further preferred case, said at least one amino acid having a side chain which does not bear a net charge at neutral pH, which is an option in accordance with the first aspect, is present and is flanked on either side by at least one, preferably at least two amino acids having a side chain which is negatively charged at neutral pH.

Noting that amino acids with side chains which do not bear a net charge at neutral pH contribute weakly to binding of said peptide to a surface comprising iron oxide, this preferred case defines an implementation of the above-mentioned (S)(Vη(S) pattern.

In a further preferred case, said peptide is capable of binding to a surface comprising iron oxide, preferably a magnetic nanoparticle.

The peptides in accordance with the first and the second aspect render the art-established application-specific functionalization of magnetic nanoparticles dispensable. The art-established functionalization of the surface of nanoparticles does not only entail the need for a specific modification of the nanoparticles for any given specific application, it also is sometimes instable or leaks. In other words, the paradigm depicted in Figure 4 does not apply to applications employing nanoparticles which make use of peptides of the present disclosure. This saves time and costs. When performing methods in accordance with the present disclosure (disclosed further below), magnetic particles can be conveniently recycled many times.

Said surface may have any geometry. It extends to planar and curved surfaces. Preferred are particles such as spherical particles including nanoparticles and spherical nanoparticles. The diameter of such particles may be in the range between 0.1 and 100 nm, preferably between 1 and 50 nm or between 1 and 20 nm including between 1 and 10 nm. Preferred particles are colloidal particles.

Preference is given to magnetic surfaces, especially magnetic nanoparticles. Magnetic nanoparticles are commercially available, e.g. from Sigma Aldrich.

The term "iron oxide" includes any form of iron (Fe) oxide such as FeO, Fe₂O₃ and Fe₃O₄. Iron oxides may also be hydrated. Preferred are magnetic iron oxides, especially Fe₃O₄.

The mentioned surface as well as the mentioned particles may consist of iron oxide or may comprise, in addition to iron oxide, other material such as oxides of other metals. Surfaces comprising mixtures of metal oxides and having magnetic properties are preferred. Preferred other metals are Co, Ni, Mn and Cu which other metals can also be comprised in small quantities only (dotation). Typical other materials are ferrites which have the following structure: XFe₂O₄ where X can be Co, Ni, Mn, Zn, Cu, Mg, Sr, Ba or mixtures of these elements.

In a preferred case, magnetic nanoparticles are prepared in accordance with the methods described in the Examples enclosed herewith.

In a preferred embodimentcase, binding to said surface, preferably to magnetic nanoparticles, occurs in (a) 50 mM Tris buffer at pH 7.4, 137 mM NaCl and 2.7 mM KCI; (b) in 50 mM MES buffer pH 6, 137 mM NaCl and 2.7 mM KCI; (c) an unbuffered solution at a pH in the range from about 5 to about 7; or (d) acetated buffered saline. The above are preferred binding conditions.

Acetate buffered saline comprises 50 mM sodium acetate buffer at pH 7, 137 mM NaCl and 2.7 mM KCI.

In a preferred case of the above-defined reversible binders, said binding is reversible in 50 mM citrate, 137 mM NaCl and 2.7 mM KCI at pH=6. These are preferred dissociation conditions. Preferably, reversible binders are characterized in that under the above-disclosed conditions at least 2/3 of bound peptide molecules are set free.

In either case, i.e. in relation to both binding conditions and dissociation conditions, it is preferred, but not required, that further agents are present. These further agents include surfactants and stabilizers. Preferred surfactants are non-ionic surfactants. Preferred non-ionic surfactants are sorbitan fatty acid esters including polyoxyethylenesorbitan fatty acid esters, such as Tween including Tween20.

In a further preferred case, (a) one, more or all of said amino acids which have a side chain which is negatively charged at neutral pH are selected from Asp and Glu; and/or (b) one, more or all amino acids, to the extent present, which have a side chain which does not bear a net charge at neutral pH are selected from Gly, Ser, Ala, Asn, Cys, Gln, His, Ile, Leu, Met, Phe, Pro, Thr, Trp, Tyr, Val and selenocystein, Gly being preferred.

Especially preferred peptides include the following:
EEEEEE (SEQ ID NO: 1)
DDDDDD (SEQ ID NO: 2)
DDDDEDDEDDDD (SEQ ID NO: 3)
DDDEDDEDDEDD (SEQ ID NO: 4)
DDDDDDDDDD (SEQ ID NO: 5)
DDDDDEDDDDDD (SEQ ID NO: 6)
DDDEDDEDDD (SEQ ID NO: 7)
DDDDDDDDDDDD (SEQ ID NO: 8)
DDEDDEDDED (SEQ ID NO: 9)
DDDDEDDDDD (SEQ ID NO: 10)
DDDDDGGDDDDD (SEQ ID NO: 11)
DDDDDDDD (SEQ ID NO: 12)
DDDDGGDDDD (SEQ ID NO: 13)
DDDDGGGGDDDD (SEQ ID NO: 14)
DDDGGGGDDD (SEQ ID NO: 15)
DDDGGDDD (SEQ ID NO: 16)
DDDGGGGGGDDD (SEQ ID NO: 17)
DDDDDDDDDDD (SEQ ID NO: 18)
EEEEGGGGEEEE (SEQ ID NO: 19)

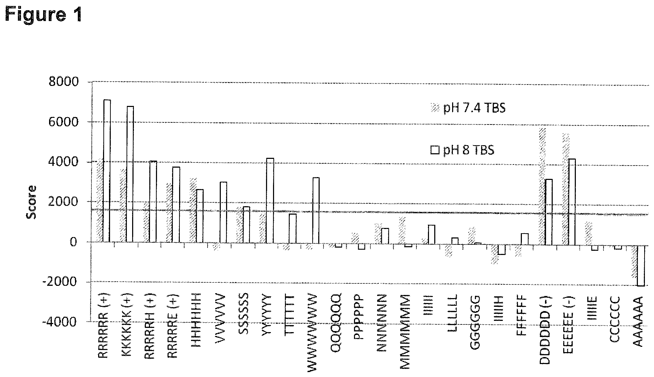

*Inter alia*, the sequences of SEQ ID NOs: 1 and 2 have been experimentally characterized; see Example 2 as well as Figure 1.

SEQ ID NO: 11 is an example of a sequence having a (S)(W)(S) pattern, wherein the first (S) region is DDDDD, the (W) region is GG, and the second (S) region is DDDDD. Further preferred specific sequences which follow the (S)(W)(S) pattern are SEQ ID NOs: 13 to 17.

In a preferred embodimentcase, increasing the length of the (W) region in a sequence having an (S)(W)(S) pattern, especially by increasing the number of Gs, while maintaining the overall number of amino acids in the two (S) regions constant, said amino acids in said (S) regions preferably being exclusively D or exclusively E, is a means of enhancing binding to surfaces containing iron oxide.

Further preferred peptides are homooligomers of D or E. Envisaged numbers of monomers in said homooligomers are 5, 6, 7, 8, 9, 10, 11, 12, 13, 14, 15, 16, 17, 18, 19, 20, 21, 22, 23, 24, 25, 26, 27, 28, 29 and 30.

In a third aspect, the present disclosure relates to a molecule which is a polypeptide or protein, said polypeptide or protein comprising at least one sequence of a peptide as defined in accordance with the first or second aspect, wherein the sequence(s) in said polypeptide or protein which do(es) not comprise said at least one sequence of a peptide are heterologous with respect to said sequence of a peptide.

It is understood that the above language "polypeptide or protein comprising at least one sequence of a peptide" relates to fusion constructs. Peptides in accordance with the present disclosure may be fused N-terminally and/or C-terminally to a polypeptide or protein. Also, it is envisaged to insert the sequence of a peptide into a polypeptide or protein such that on either side of the peptide there are flanking sequences, said flanking sequences originating from said polypeptide or protein. As mentioned above, the advantage of fusion constructs is that they can be obtained by transcription and translation of nucleic acids. For such molecule to be encoded by a nucleic acid, it is understood that polypeptide or protein as well as peptide consist exclusively of amino acids which can be encoded by a nucleic acid. Typically, these are the 20 proteinogenic amino acids.

Accordingly, the present disclosure also provides relates to a nucleic acid encoding a peptide of the first or second aspect or a molecule in accordance with the third aspect The nucleic acid may consist of a nucleotide sequence encoding said peptide or said molecule or may comprise such coding sequence and furthermore comprise flanking sequences. A preferred implementation of a nucleic acid comprising also flanking sequences is an expression vector such as a pET vector. In addition to a vector which contains a nucleic acid sequence encoding a molecule or fusion construct of the disclosure, also expression vectors are provided which contain one or more nucleic acid sequences encoding a peptide in accordance with the first or second aspect and a cleavage site for a restriction enzyme. Such cleavage site permits the insertion of a nucleic acid encoding a polypeptide or protein of interest.

It is apparent from the definition of the molecule in accordance with the third aspect that naturally occurring polypeptides and proteins do not fall under the term "molecule" Instead, there is the requirement that the peptide on the one hand and the above-mentioned flanking sequences on the other hand are heterologous to each other. Similarly, the sequence of said polypeptide or protein as a whole is required to be heterologous with respect to the sequence of the peptide under consideration in accordance with the first or second aspect.

It is furthermore envisaged to use linker sequences which connect the peptide in accordance with the first or second aspect to a polypeptide or protein or, to the extent the sequence of said peptide is inserted into said polypeptide or protein, to a partial sequence of said polypeptide or protein. Preferably, such linker sequences, to the extent they are present, are heterologous with regard to said polypeptide or protein and furthermore heterologous with regard to the sequence of said peptide in accordance with the first or second aspect. Preferred linker sequences include oligoglycine sequences and the sequence SSG.

In a fourth aspect, the present disclosure relates to a molecule covalently bound to at least one sequence of a peptide in accordance with the first aspect (or in accordance with the second aspect), wherein the covalent bond is not a main chain peptide bond, wherein said molecule is preferably a polypeptide or protein or a nucleic acid such as DNA or RNA including siRNAs. Further envisaged molecules are organic molecules including small organic molecules with a molecular weight between 100 and 1000 Da and/or pharmaceuticals which are organic molecules; ligands including ligands of naturally occurring receptors; and toxins or toxoids.

This aspect relates to conjugates. Conjugates are herein distinguished from fusion constructs. Accordingly, the moiety which is conjugated to a peptide in accordance with the present disclosure may be a polypeptide or protein, but does not have to. The moiety to be conjugated may be conjugated to the N-terminus of the peptide. Ethyl-dimethylaminopropyl-carbodiimid (EDC) coupling may be used.

In preferred cases of the molecule in accordance with the third and the fourth aspect, said polypeptide or protein is (a) an antibody or fragment thereof; or (b) an enzyme or hormone.

In a fifth aspect, the present disclosure relates to a surface comprising iron oxide, preferably a magnetic nanoparticle, which is bound to at least one peptide or molecule as defined in accordance with the first, second, third or fourth aspect.

The present disclosure also relates to uses of the surface in accordance with the fifth aspect, said uses being for a biotechnological process. In a preferred case of said use, said molecule is an enzyme and said biotechnological process is or comprises a reaction catalyzed by said enzyme.

Related thereto, the present disclosure also relates to a method for enzymatic processing of a substrate, said method comprising: (a) bringing into a contact a surface in accordance with the fifth aspect, wherein said molecule is an enzyme, with a substrate of said enzyme. Preferably, said contacting is effected under conditions that permit conversion of said substrate into a product, the conversion being catalyzed by said enzyme. Such conditions are generally known in the art and may be specific for a given enzyme/substrate pair.

In a preferred case of said method, said method further comprises one or more of the following: (b) separating said surface from the reaction mixture; (c) enriching or purifying the obtained product.

In a sixth aspect, the present disclosure relate to a method of separating a molecule as defined in accordance with the third or fourth aspect from a sample, said method comprising: (a) contacting said sample with a surface comprising iron oxide, preferably magnetic nanoparticles, under conditions allowing binding of said molecule to said surface; and (b) separating said surface from said sample; thereby separating said molecule from said sample.

Given that the present disclosure relates to peptides as well as molecules which are capable of binding to a surface-containing iron oxide, the skilled person can determine without further ado the conditions in accordance with item (a) of this aspect. Preferred conditions are disclosed further below.

Separating in accordance with step (b) of the method of the sixth aspect can be done by any means. To the extent magnetic surfaces, magnetic particles and magnetic nanoparticles are used, said separating is preferably effected with a magnet.

Devices for separating magnetic particles from mixtures are available from various manufacturers including Thermo Fisher.

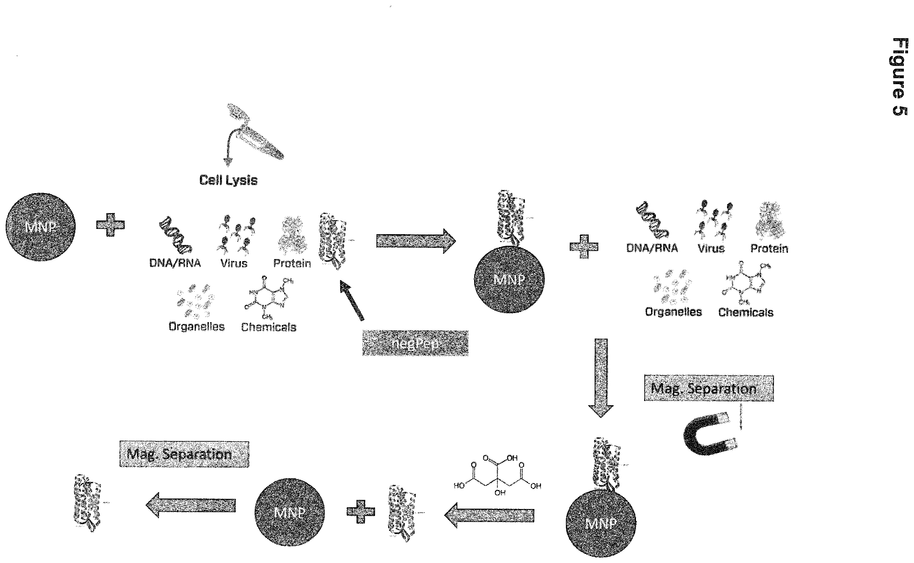

Figure 5 illustrates an exemplary manner the method in accordance with the sixth aspect.

In a seventh aspect, the present disclosure relates to a method of separating an analyte from a sample, said analyte being capable of binding to a molecule in accordance with the third or fourth aspect, said method comprising: (a) bringing into contact said sample, said molecule and a surface comprising iron oxide, preferably magnetic nanoparticles, under conditions allowing (i) binding of said molecule to said surface and (ii) binding of said analyte to said molecule; and (b) separating said surface from said sample; thereby separating said analyte from said sample.

As noted above, the skilled person, provided with the information of the present disclosure, can determine without further ado the conditions in accordance with item (i) of step (a) of the method of the seventh aspect.

Conditions in accordance with item (ii) are generally known in the art. To give an example, if said analyte is an antigen or a molecule comprising an epitope recognized by a given antibody, and said molecule is an antibody, the antibody in turn comprising a peptide in accordance with the first or second aspect, the known conditions which allow binding of the epitope to the antibody define the conditions in accordance with item (ii). Fine-tuning and/or optimizing such conditions can be done by the skilled person without further ado, for example by slightly varying conditions and determining the performance upon such variation.

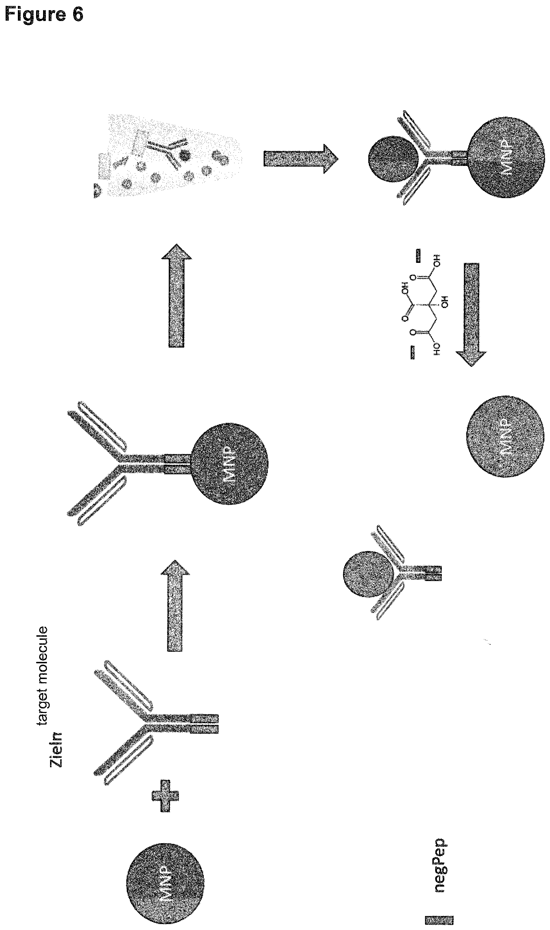

The method in accordance with the seventh aspect is illustrated in Figure 6.

In a preferred case, step (a) is implemented by (a)(i) bringing into contact said molecule and a surface comprising iron oxide, preferably magnetic nanoparticles, followed by (a)(ii) bringing into contact the result of (a)(i) with said sample.

In a preferred case of the methods of the present disclosure, these methods further comprise (c) washing said surface; and/or (d)(i) dissociating said molecule from said surface; (ii) optionally followed by removing said surface from the result of (i).

In a preferred case, said dissociating in step (d)(i) is performed by adding (a) a carboxylic acid, preferably a carboxylic acid comprising two or three carboxylic groups, most preferably citric acid, said carboxylic acid preferably being in buffered solution, most preferably under the following conditions: 50 mM citrate, 137 mM NaCl and 2.7 mM KCI at pH=6; and/or (b) an inorganic oxo acid, preferably phosphoric acid, said oxo acid preferably being in buffered solution.

A further preferred carboxylic acid is tartaric acid. A further preferred carboxylic acid is oxalic acid.

In a further preferred case of the method in accordance with the seventh aspect, the method further comprises (e) dissociating said analyte from said molecule.

Since the interaction between analyte and molecule will generally be known, also conditions suitable for said dissociating are known to the skilled person or can be determined without further ado.

Related to the seventh aspect, the present disclosure also relates to, in an eighth aspect, a method of determining presence or absence of an analyte in a sample suspected of comprising said analyte, said method comprising steps (a) and (b) as defined in relation to a seventh aspect, optionally one, more or all of steps (c), (d)(i), (d)(ii) and (e) as defined in preferred cases herein above, thereby obtaining a mixture, and (f) analyzing said mixture for the presence of said analyte.

Said analyzing can be performed with any known analytic method. Preferred methods include mass spectrometry and liquid chromatography such as HPLC.

In a preferred case of the methods in accordance with the sixth, seventh and eighth aspect, said conditions in step (a) are (a) Tris buffer at a pH between about 7 and about 8; (b) MES buffer at a pH between about 5.8 and about 6.5; or (c) an unbuffered solution at a pH in the range from about 5 to about 7; or (d) acetate buffered saline, and preferably as defined in relation to binding of peptides of the first aspect to a surface comprising iron oxide. Any of said conditions preferably include a temperature in the interval between 18 and 35°C, more preferably 20°C or room temperature.

Further suitable conditions include HEPES buffer, HEPPS buffer, MOPS buffer, TES buffer, TAPS buffer and PIPES buffer, each of them buffers having a pH within plus or minus 1 of the p*K*ₐ value of the respective buffer substance.

In a ninth aspect, the present disclosure relates to a kit comprising or consisting of (a)(i) a peptide in accordance with the first aspect; (ii) a molecule in accordance with the third or fourth aspect; and/or (iii) a nucleic acid encoding a peptide of (i) or a molecule of (ii), wherein said molecule is a polypeptide or protein, wherein said nucleic acid is preferably comprised in an expression vector.

In a preferred case, the kit comprises one or more or all of the following (b) a surface comprising iron oxide, preferably magnetic nanoparticles; (c) a first solution or constituents for preparing said first solution, said first solution being capable of establishing conditions which allow binding of said peptide and/or said molecule to said surface; (d) a second solution or constituents for preparing said second solution, said second solution being capable of establishing conditions which allow dissociating said peptide and/or said molecule from said surface; and (e) instructions for use of said kit, preferably for performing a method of the present disclosure.

The Figures show:
- **Figure 1:**: Binding scores of magnetic nanoparticles on peptides at pH 7.4 and pH 8 in Tris buffered saline with 0.25% Tween 20 (T-TBS). The particle suspensions were disaggregated in an ultrasound bath for 15 min before incubation with the membrane. The results are the averages of two membranes tested in the same experiment. The horizontal line indicates the noise level in this experiment.

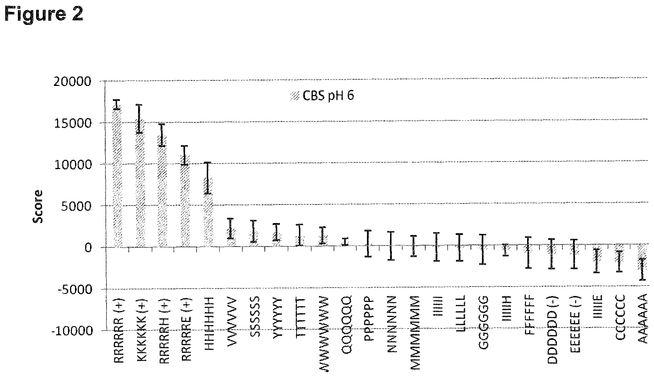

- **Figure 2:**: Binding affinities of magnetic nanoparticles to peptides in Citrate buffered saline pH 6 with 0.25% Tween 20 (T-CBS). The particle suspensions were disaggregated in an ultrasound bath for 15 min before incubation with the membrane. The horizontal line stands for the background noise level.

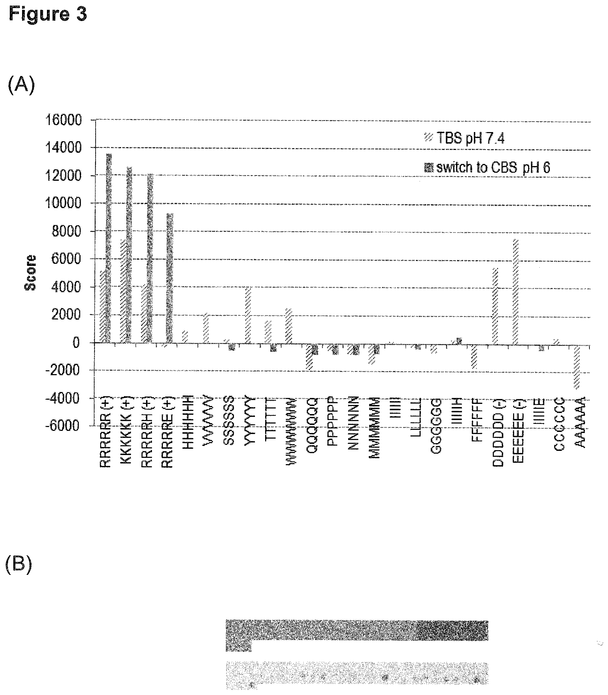

- **Figure 3:**: Binding scores of MNPs on peptide spots after incubation in T-TBS pH 7.4 for 1 h and after transfer of membrane to T-CBS pH 6. (A) The noise level is represented by the horizontal line. (B) Image of a membrane with spots that bound to MNPs being marked.
- **Figure 4:**: In accordance with art-established methods, prior to any application, magnetic nanoparticles have to be functionalized in a tailored manner. In the figure, functionalization of the nanoparticle requires coating thereof with the molecules displayed in dark grey.
- **Figure 5:**: Purification of polypeptides or proteins tagged with peptides of the present disclosure with magnetic nanoparticles. Peptides of the present disclosure are fused to the polypeptide or protein of interest and MNPs are added to the initial mixture. MNPs bind to the polypeptide or protein and can be separated by using a magnet. Upon changing the conditions, the polypeptide or protein can be separated from the magnetic nanoparticles. The magnetic nanoparticles in turn can be separated and re-used again.
- **Figure 6:**: An antibody having peptides in accordance with the present disclosure fused to its F_{C} portion binds to magnetic nanoparticles. Antibody-loaded nanoparticles can be used to separate molecules comprising the cognate epitope from a mixture. When peptides in accordance with the present disclosure are used which bind reversible to magnetic nanoparticles, the antibody-antigen complex can be detached from the magnetic nanoparticle by changing the conditions. As in the preceding figures, magnetic nanoparticles can be re-used.
- **Figure 7:**: Interaction data for peptides on a PEPperCHIP^{®}-Array with magnetic nanoparticles in ddH₂O at pH 4. Data are averages for duplicates of the same experiment.
- **Figure 8:**: Scores for the binding of peptides to magnetic nanoparticles in Tris buffered saline (T-TBS) at pH 7.4 on cellulose membranes from Intavis. The data represent averages of two experiments.
- **Figure 9:**: Adsorption isotherms of octa-glutamic acid on magnetite nanoparticles (1 g L⁻¹) at different pH (top) and in different buffers at pH 7 (bottom), all without Tween. Adsorption isotherms at pH 4.5 and 7 are fitted with a Langmuir function, while the isotherms at pH 9 and 10 are fitted with a Freundlich function.
- **Figure 10:**: Adsorption isotherms of tagged green fluorescent protein (GFP) on magnetic nanoparticles in Tris buffered saline (TBS) at pH 7 with 0.1% Tween (T-TBS).
- **Figure 11:**: Magnetic separation of Glu₆-tagged protein from bacterial lysate in TBS at pH 7 using a high gradient magnetic separator. Elution has been effected with citrate buffered saline (CBS) at pH 7.

The Examples illustrate the invention.

### Example 1

### Materials and Methods

### Synthesis of magnetic nanoparticles

The iron oxide nanoparticles used for this study were synthesized by co-precipitation of Fe²⁺ and Fe³⁺ in alkaline aqueous solutions adapted to the procedure described by Roth *et al.* (Roth, H.-C., et al. (2015): Influencing factors in the CO-precipitation process of superparamagnetic iron oxide nano particles: A model based study, Journal of Magnetism and Magnetic Materials 377, pp. 81-89). Briefly, 21.2 g of FeCl₃ x 6 H₂O and 8.29 g of FeCl₂ x 4 H₂O were dissolved in 200 mL of deionized, degassed water which equals a Fe(lll) : Fe(ll) ratio of 1.88 : 1. This iron chloride solution was added to 1 L of a 1 M solution of NaOH prepared with deionized, degassed water stirring at 250 rpm in a reaction vessel. The reaction mixture was kept under nitrogen atmosphere at 25°C and stirred for 30 more minutes before the nanoparticles that had formed were washed several times with deionized water until the conductivity of the MNP solution was below 200 µS cm⁻¹. In order to separate the particles from the washing water the mixture was placed on a NdFeB permanent magnet. FeCl₃ x 6 H₂O and sodium hydroxide were purchased from AppliChem GmbH, Germany in the highest purity available. FeCl₂ x 4 H₂O extra pure was obtained from Merck KGaA, Germany.

### Zetapotential measurements

For zetapotential measurements, a suspension of 0.4 g/L magnetic nanoparticles in buffer was sonicated for 15 minutes. In a Beckman Coulter Delsa Nano C, the zetapotential was determined at 25°C three times with 10 accumulation times at 5 different positions in a flow cell each at 60 V with a pinhole of 50 µm.

### Magnetic nanoparticle binding assay

In order to determine the binding between peptides and magnetic nanoparticles (MNPs) CelluSpot peptide arrays from Intavis with 5 to 10 nmol of peptides per spot were used. The cellulose membrane on which the peptides had been synthesized by the manufacturer was conditioned with 1 mL of methanol in order to rehydrate hydrophobic peptides (Golemis, E. and Adams, P. D. (2005): Protein-protein interactions. A molecular cloning manual. 2nd ed. Cold Spring Harbor, N.Y.: Cold Spring Harbor Laboratory Press). The buffers employed were T-CBS, T-PBS or T-TBS which is 50 mM citrate, phosphate or tris, respectively, with 0.25% Tween 20, 137 mM NaCl and 2.7 mM KCI. The orbital shaker used for incubation was MulitBio3D from Biosan.

The array was washed three times for 10 min each with 50 mL of buffer. After washing, the membrane was incubated for 60 minutes in a MNP solution (Kuboyama, M., et al. (2012): Screening for silver nanoparticle-binding peptides by using a peptide array, Biochemical Engineering Journal 66 (0), pp. 73-77). The spots on which magnetite bound became dark grey. In order to remove unbound particles the membrane was washed three times for 10 minutes each with buffer (Golemis, E. and Adams, P. D. (2005): Protein-protein interactions. A molecular cloning manual. 2nd ed. Cold Spring Harbor, N.Y.: Cold Spring Harbor Laboratory Press). Then the cellulose membrane was dried overnight at 4°C and an image of it was taken using a GelDoku station.

### Peptidarray-Experimente

The PEPperCHIP^{®}-Array with printed peptides has been purchased from PEPperPRINT Germany. 0.4 g/L MNP suspended in ddH₂O pH 4 have been desagglomerated by ultrasonication for 4 minutes with a Branson sonifier 450D prior to a 1:10 dilution with ddH₂O pH 4. This suspension was incubated on the chip with an orbital shaker for 1 h and then dipped into 1 mM Tris buffer pH 7.4. The chip was scanned with a Typhoon FLA 9500 scanner in digitalization mode with a resolution of 10 µm and analyzed with the software PepSlideAnalyzer.

### Adsorption isotherms with peptides

The octa-glutamic acid peptide (101 mg) was obtained from metabion international AG (Germany) as a lyophilized powder with a purity of >95% and stored at +4 °C prior to use. For the binding experiments different amounts of peptides were incubated with magnetite nanoparticles (1 g L⁻¹) at different pH and buffer conditions for at least one hour at 25°C under vigorous shaking. The supernatant was separated from the particles with hand magnets, decanted and analysed with an Infinite M200 Microplate Reader (Tecan Deutschland, Germany) at 230 nm.

### Adsorption isotherms with proteins

0.5 mL protein solution was mixed with a magnetite particle solution 1 g L⁻¹ in a 1.5 mL Eppendorf vessel and incubated for 1 h at 25°C and 1000 rpm in a thermomixer comfort from Eppendorf.

The particles and the supernatant were separated with a NdFeB magnet. The supernatant was centrifuged at 17000 x g at 4 °C for 5 minutes in order to remove residual particles prior to analysis.

### High-gradient magnetic separation

The process starts with the incubation of 0.5 L cell lysate in TBS pH 7 containing Glu₆-GFP with 0.5 L magnetite (2 g/L) in a feed tank. This mixture is stirred at ~900 rpm for 1 h at room temperature. Every 15 min aliquots of 2 mL are taken in order to monitor the adsorption.

After the incubation, the suspension is pumped through a magnetic separation chamber as described (Roth et al. A High-Gradient Magnetic Separator for Highly Viscous Process Liquors in Industrial Biotechnology, Chemical Engineering and Technology 39 (3) 469-476). The supernatant containing impurities and unbound protein is removed by two washing steps, each 0.5 L containing TBS pH 7. The flow direction through the chamber is changed every 200 s during the washing procedure. During the washing procedure 10 samples are taken of the outlet fractions. For the elution citrate buffered saline (CBS) at pH 7 is used which is provided in the feed tank. The particles are resuspended as the magnetic field is removed and the whole mixture is circulated through the whole apparatus with changing flow directions every 15 minutes. Aliquots are taken each 10 minutes and after 1 h the suspension is pumped through the magnetic field again in order to separate the protein from the particles. In a second step the resuspension is improved by the addition of a two phase flow where air is mixed with the buffer for 10 minutes as described (Roth et al. A High-Gradient Magnetic Separator for Highly Viscous Process Liquors in Industrial Biotechnology, Chemical Engineering and Technology 39 (3) 469-476). Aside from the two phase flow through the system, the process is similar to the first elution step.

### Example 2

### Peptide arrays

The binding behavior of magnetite nanoparticles (MNPs) to peptides was investigated using a peptide array. Figure 1 shows that at a pH of 7.4 in Tris buffered saline, the negatively charged peptides hexa-glutamic acid (6E) and hexa-aspartic acid (6D) bound strongest to the MNPs. The second highest scores at pH 7.4 were achieved by the positive peptides hexa-arginine (6R), hexa-lysine (6K), 5RH and 5RE as well as hexa-histidine (6H) which is nearly neutral at a pH of 7.4 according to the theoretical pl of 7.21 determined by the ProtParam Tool by the Bioinformatics Resource Portal ExPASy (Bjellqvist, B., et al. (1993): The focusing positions of polypeptides in immobilized pH gradients can be predicted from their amino acid sequences, Electrophoresis 14 (1), pp. 1023-1031). It is in accordance with literature that histidine residues can form complexes with metals and that carboxy groups bind them through electrostatic interaction (Kozlowski, H., et al. (2013): Specific metal ion binding sites in unstructured regions of proteins, Coordination Chemistry Reviews 257 (19-20), pp. 2625-2638). The MNP surface was shown to be slightly positively charged in zetapotential measurements at a pH of 7.4 as shown in Table 1. The point of zero charge was determined to be around 7.8 by acidometry. Hydrophobic peptides do not show any binding while the binding of polar peptides is low to moderate.

**Table 1: Zetapotential of magnetic nanoparticles in different buffers and at different pHs**

| Buffer | pH | Zetapotential, mV |
|---|---|---|
| T-TBS | 7.4 | +3.7 |
| | 8 | |
| T-PBS | 6 | -27.8 |
| | 7.4 | |
| | 8 | |
| T-CBS | 6 | -34.74 |

### Example 3

### Comparison of homooligomers with heterooligomers

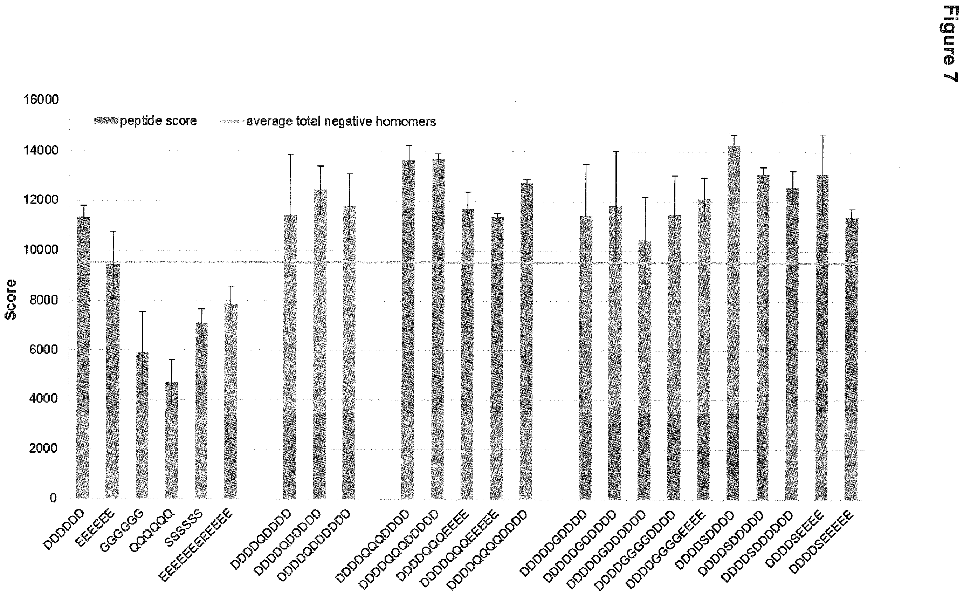

As demonstrated in Figure 7, heteromeric peptides with interspersed serine, glycine or glutamine residues exhibit better binding than homooligomers of glutamine or asparagines. The heteromers have a length between 9 and 12 amino acids and the fraction of residues which are negatively charged at neutral pH is at least 2/3.

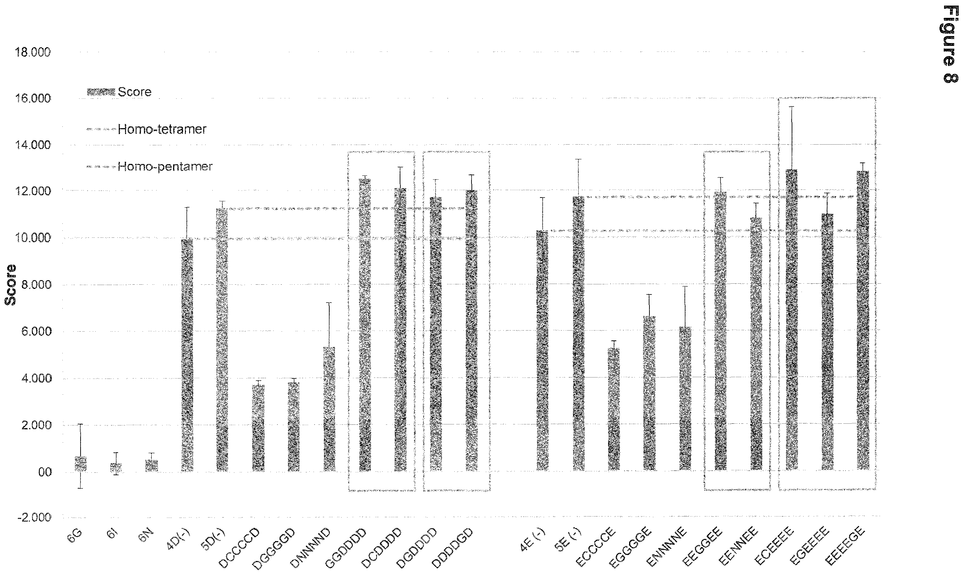

As shown in Figure 8, such results could be confirmed on a different array format. In particular, to the extent the fraction of cysteine, glycine or asparagines is above 1/3, binding drops below the binding of the corresponding homooligomers. On the other hand, if the fraction of cysteine, glycine or asparagines does not exceed 1/3, binding is increased as compared to the corresponding homooligomers.

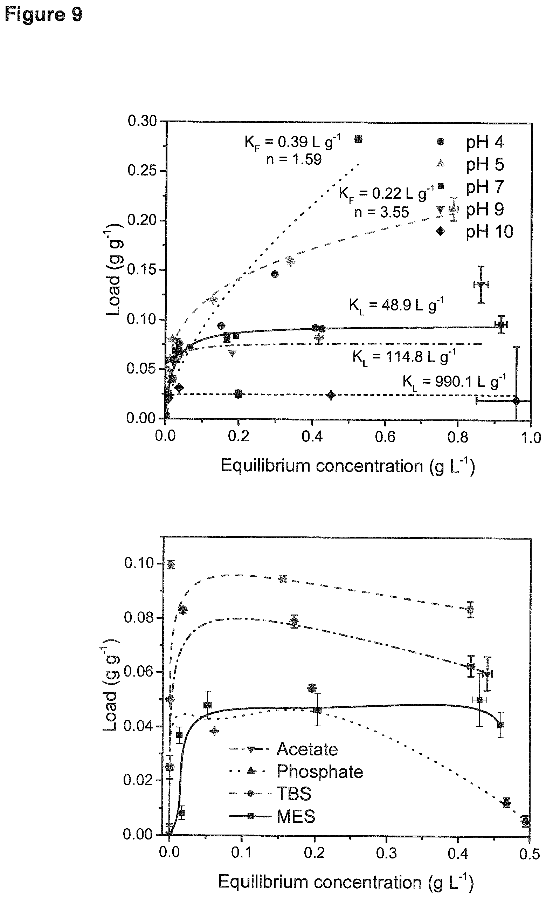

Figure 9 shows affinity constants of peptides in accordance with the present disclosure to magnetic nanoparticles in different buffers and at different pH values.

### Example 4

### Separation method

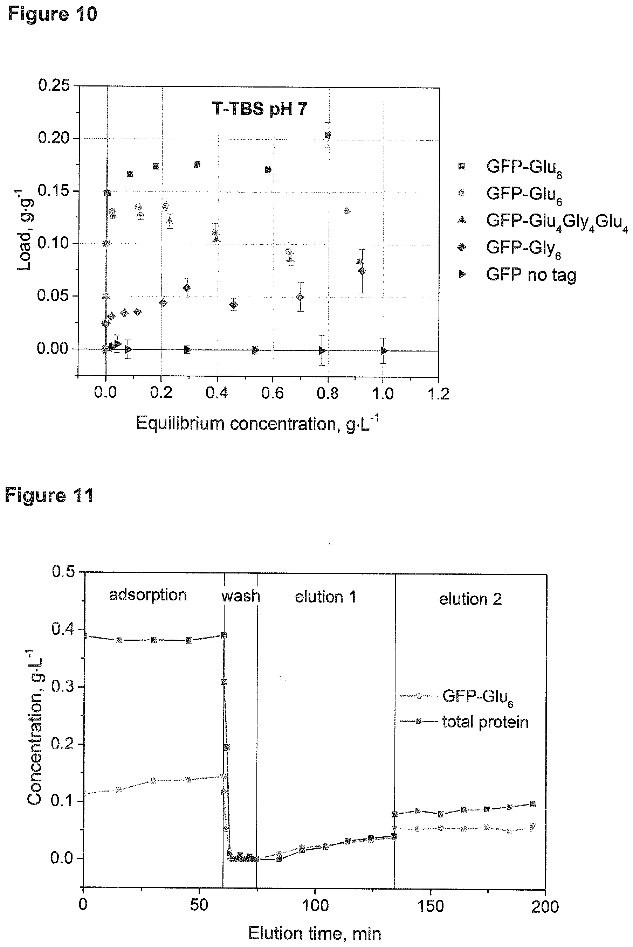

The data depicted in Figure 10 demonstrate that peptides in accordance with the present disclosure when used in proteins to be purified allow binding to magnetic nanoparticles. In a tris buffered system, Glu₆-, Glus-, and Glu₄Gly₄Glu₄-tagged protein binds with high affinity and high maximal loads as compared to untagged protein; see also Table 2 below.

**Table 2: Binding constants of tagged proteins to MNPs in T-TBS at pH 7.**

| Protein | qₘₐₓ, g·g⁻¹ | K_{D}, g·L⁻¹ |
|---|---|---|
| GFP-Glu₈ | 0.179 ± 0.006 | 4E-04 ± 2E-04 |
| GFP-Glu₆ | 0.120 ± 0.008 | 4E-15 ± 3E-03 |
| GFP-E₄G₄E₄ | 0.102 ± 0.011 | 7E-10 ± 6E-03 |
| GFP-Gly₆ | 0.047± 0.005 | 1E-03± 1E-03 |
| GFP no tag | 8E-05±1E-03 | 2E-16 ± 6E-01 |

Using a magnetic separator (as described in Roth et al., Chem. Eng. Technol. 2016, 39, 469-476) has been used to separate GFP tagged with a peptide in accordance with the disclosure (Gluₓ) from a bacterial broth using magnetic particles in TBS. CBS has been used as elution buffer. Figure 11 displays the concentration of the protein of interest (GFP-Glu₆) and the total protein concentration as a function of time.

## Claims

1. A peptide consisting of a sequence of 5 to 30, preferably 6 to 12, most preferably 10 to 12 amino acids, wherein said peptide is capable of binding to a surface comprising magnetic iron oxide, and wherein
(a) at least 2/3 of said amino acids are selected from Asp and Glu; and
(b) at least one amino acid which has a side chain which does not bear a net charge at neutral pH is present, selected from Gly, Ser, Ala, Asn, Cys, Gln, His, Ile, Leu, Met, Phe, Pro, Thr, Trp, Tyr, Val and selenocystein; and flanked on either side by at least one, preferably at least two amino acids as defined in (a).

2. The peptide of claim 1, wherein the fraction of amino acids which have a side chain which is positively charged at neutral pH is
in the range between zero and 0.2, preferably zero.

3. The peptide of claim 1 or 2, wherein said peptide is capable of binding to a magnetic nanoparticle.

4. The peptide of any one of claims 1 to 3, wherein one, more or all amino acids of claim 1(b) are Gly.

5. A molecule which is a polypeptide or protein, said polypeptide or protein being a fusion construct and comprising at least one sequence of a peptide
(i) as defined in any one of the preceding claims; or
(ii) consisting of a sequence of 5 to 30, preferably 6 to 12, most preferably 10 to 12 amino acids, wherein said peptide is capable of binding to a surface comprising magnetic iron oxide, and wherein
(a) at least 2/3 of said amino acids are selected from Asp and Glu;
(b) at least one amino acid which has a side chain which does not bear a net charge at neutral pH is present, selected from Gly, Ser, Ala, Asn, Cys, Gln, His, Ile, Leu, Met, Phe, Pro, Thr, Trp, Tyr, Val and selenocystein,
wherein the sequence(s) in said polypeptide or protein which do(es) not comprise said at least one sequence of a peptide are heterologous with respect to said sequence of a peptide.

6. A molecule covalently bound to at least one sequence of a peptide as defined in any one of claims 1 to 5, wherein the covalent bond is not a main chain peptide bond, wherein said molecule is a polypeptide or protein or a nucleic acid such as DNA or RNA including siRNAs.

7. A surface comprising iron oxide, preferably a magnetic nanoparticle, which is bound to at least one peptide or molecule as defined in any one of the preceding claims.

8. A method of separating a molecule as defined in claim 5 or 6 from a sample, said method comprising:
(a) contacting said sample with a surface comprising iron oxide, preferably magnetic nanoparticles, under conditions allowing binding of said molecule to said surface; and
(b) separating said surface from said sample;
thereby separating said molecule from said sample.

9. A method of separating an analyte from a sample, said analyte being capable of binding to a molecule as defined in claim 5 or 6, said method comprising:
(a) bringing into contact said sample, said molecule and a surface comprising iron oxide, preferably magnetic nanoparticles, under conditions allowing (i) binding of said molecule to said surface and (ii) binding of said analyte to said molecule; and
(b) separating said surface from said sample;
thereby separating said analyte from said sample.

10. The method of claim 8 or 9, further comprising
(c) washing said surface; and/or
(d)
(i) dissociating said molecule from said surface;
(ii) optionally followed by removing said surface from the result of (i).

11. The method of claim 10, wherein said dissociating in step (d)(i) is performed by adding
(a) a carboxylic acid, preferably a carboxylic acid comprising two or three carboxylic groups, most preferably citric acid, said carboxylic acid preferably being in buffered solution; and/or
(b) an inorganic oxo acid, preferably phosphoric acid, said oxo acid preferably being in buffered solution.

12. A method of determining presence or absence of an analyte in a sample suspected of comprising said analyte, said analyte being capable of binding to a molecule as defined in claim 5 or 6, said method comprising steps (a) and (b) as defined in claim 9,
optionally one, more or all of steps (c), (d)(i), (d)(ii) and (e) as defined in claims 10 and 11,
thereby obtaining a mixture, and
(f) analyzing said mixture for the presence of said analyte.

13. A kit comprising or consisting of
(a)
(i) a peptide as defined in any one of claims 1 to 4;
(ii) a molecule as defined in claim 5 or 6; and/or
(iii) a nucleic acid encoding a peptide of (i) or a molecule of (ii), wherein said molecule is a polypeptide or protein, wherein said nucleic acid is preferably comprised in an expression vector.

14. The kit of claim 13, further comprising one, more or all of the following:
(b) a surface comprising iron oxide, preferably magnetic nanoparticles;
(c) a first solution or constituents for preparing said first solution, said first solution being capable of establishing conditions which allow binding of said peptide and/or said molecule to said surface;
(d) a second solution or constituents for preparing said second solution, said second solution being capable of establishing conditions which allow dissociating said peptide and/or said molecule from said surface; and
(e) instructions for use of said kit, preferably for performing a method as defined in any one of claims 8 to 12.

## Patentansprüche

1. Peptid bestehend aus einer Sequenz von 5 bis 30, bevorzugt 6 bis 12, besonders bevorzugt 10 bis 12 Aminosäuren, wobei das Peptid an eine magnetisches Eisenoxid enthaltende Oberfläche binden kann, und wobei
(a) mindestens 2/3 der Aminosäuren ausgewählt sind aus Asp und Glu; und
(b) mindestens eine Aminosäure vorhanden ist, welche eine Seitenkette hat, die keine Nettoladung bei neutralem pH trägt, ausgewählt von Gly, Ser, Ala, Asn, Cys, Gln, His, Ile, Leu, Met, Phe, Pro, Thr, Trp, Tyr, Val und Selenocystein; und auf jeder Seite von mindestens einer, bevorzugt mindestens zwei Aminosäuren wie in (a) definiert, flankiert ist.

2. Peptid nach Anspruch 1, wobei der Anteil der Aminosäuren, welche eine Seitenkette haben, die bei neutralem pH positiv geladen ist, in dem Bereich zwischen null und 0,2, bevorzugt null ist.

3. Peptid nach Anspruch 1 oder 2, wobei das Peptid an ein magnetisches Nanopartikel binden kann.

4. Peptid nach einem der Ansprüche 1 bis 3, wobei eine, mehrere oder alle Aminosäuren nach Anspruch 1(b) Gly sind.

5. Molekül, welches ein Polypeptid oder Protein ist, wobei das Polypeptid oder Protein ein Fusionskonstrukt ist und mindestens eine Sequenz eines Peptids umfasst,
(i) wie in einem der vorigen Ansprüche definiert; oder
(ii) bestehend aus einer Sequenz von 5 bis 30, bevorzugt 6 bis 12, besonders bevorzugt 10 bis 12 Aminosäuren, wobei das Peptid an eine magnetisches Eisenoxid enthaltende Oberfläche binden kann, und wobei
(a) mindestens 2/3 der Aminosäuren ausgewählt sind aus Asp und Glu;
(b) mindestens eine Aminosäure vorhanden ist, welche eine Seitenkette hat, die keine Nettoladung bei neutralem pH trägt, ausgewählt von Gly, Ser, Ala, Asn, Cys, Gln, His, Ile, Leu, Met, Phe, Pro, Thr, Trp, Tyr, Val und Selenocystein;
wobei die Sequenz(en) in dem Polypeptid oder Protein, welche nicht mindestens eine Sequenz eines Peptids umfasst, heterolog sind gegenüber der Sequenz eines Peptids.

6. Molekül kovalent gebunden an mindestens eine Sequenz eines Peptids wie in einem der Ansprüche 1 bis 5 definiert, wobei die kovalente Bindung nicht eine Hauptkettenpeptidbindung ist, wobei das Molekül ein Polypeptid oder Protein oder eine Nukleinsäure, wie DNA oder RNA einschließlich siRNAs, ist.

7. Oberfläche umfassend Eisenoxid, bevorzugt ein magnetisches Nanopartikel, welches an mindestens ein Peptid oder Molekül wie in einem der vorigen Ansprüche definiert, gebunden ist.

8. Verfahren zum Abtrennen eines Moleküls wie in Anspruch 5 oder 6 definiert von einer Probe, wobei das Verfahren umfasst:
(a) in Kontakt bringen der Probe mit einer Eisenoxid-, bevorzugt magnetische Nanopartikel, enthaltenden Oberfläche unter Bedingungen, die eine Binding des Moleküls an die Oberfläche erlauben; und
(b) Abtrennen der Oberfläche von der Probe;
wodurch das Molekül von der Probe abgetrennt wird.

9. Verfahren zum Abtrennen eines Analyten von einer Probe, wobei der Analyt an ein Molekül wie in Anspruch 5 oder 6 definiert binden kann, wobei das Verfahren umfasst:
(a) in Kontakt bringen der Probe, des Moleküls und der Eisenoxid-, bevorzugt magnetische Nanopartikel, enthaltenden Oberfläche unter Bedingungen die (i) die Bindung von dem Molekül an die Oberfläche und (ii) die Bindung des Analyten an das Molekül erlauben; und
(b) Abtrennen der Oberfläche von der Probe;
wodurch der Analyt von der Probe abgetrennt wird.

10. Verfahren nach Anspruch 8 oder 9, ferner umfassend:
(c) Waschen der Oberfläche; und/oder
(d)
(i) Ablösen des Moleküls von der Oberfläche
(ii) gegebenenfalls gefolgt von Entfernen der Oberfläche von dem Ergebnis von (i).

11. Verfahren nach Anspruch 10, wobei das Ablösen in Schritt (d)(i) ausgeführt wird durch Zugabe von:
(a) einer Carbonsäure, bevorzugt einer Carbonsäure die zwei oder drei Carboxylgruppen umfasst, besonders bevorzugt Zitronensäure, wobei die Carbonsäure bevorzugt in einer gepufferten Lösung vorliegt; und/oder
(b) eine anorganische Oxosäure, bevorzugt Phosphorsäure, wobei die Oxosäure bevorzugt in einer gepufferten Lösung vorliegt.

12. Verfahren zur Bestimmung des Vorhandenseins oder Fehlens eines Analyten in einer Probe, die vermutlich den Analyten enthält, wobei der Analyt an ein Molekül wie in Anspruch 5 oder 6 definiert binden kann, wobei das Verfahren Schritte (a) und (b) wie in Anspruch 9 definiert umfasst, gegebenenfalls einen, mehrere oder alle Schritte (c), (d)(i), (d)(ii) und (e) wie in den Ansprüchen 10 und 11 definiert, wobei dadurch ein Gemisch erhalten wird, und
(f) das Gemisch auf das Vorhandensein des Analyten geprüft wird.

13. Kit umfassend oder bestehend aus:
(a)
(i) einem Peptid wie in einem der Ansprüche 1 bis 4 definiert;
(ii) einem Molekül wie in Anspruch 5 oder 6 definiert; und/oder
(ii) einer Nukleinsäure, die ein Peptid nach (i) oder ein Molekül nach (ii) kodiert, wobei das Molekül ein Polypeptid oder Protein ist, wobei die Nukleinsäure bevorzugt einem Expressionsvektor enthalten ist.

14. Kit nach Anspruch 13, ferner umfassend ein, mehrere oder jedes der Folgenden:
(b) eine Eisenoxid-, bevorzugt magnetische Nanopartikel, enthaltende Oberfläche;
(c) eine erste Lösung oder Bestandteile zum Ansetzen der ersten Lösung, wobei die erste Lösung Bedingungen schaffen kann, welche die Bindung von dem Peptid und/oder dem Molekül zu der Oberfläche erlauben;
(d) eine zweite Lösung oder Bestandteile zum Ansetzen der zweiten Lösung, wobei die zweite Lösung Bedingungen schaffen kann, die das Ablösen des Peptids und/oder des Moleküls von der Oberfläche erlauben; und
(e) Anweisungen zur Verwendung des Kits, bevorzugt zur Durchführung eines Verfahrens wie in einem der Ansprüche 8 bis 12 definiert.

## Revendications

1. Peptide consistant en une séquence de 5 à 30, de préférence 6 à 12, tout spécialement 10 à 12 acides aminés, lequel peptide est capable de se lier à une surface comprenant de l'oxyde de fer magnétique, et dans lequel
(a) au moins 2/3 desdits acides aminés sont choisis parmi Asp et Glu ; et
(b) au moins un acide aminé qui a une chaîne latérale qui ne porte pas une charge nette à un pH neutre est présent, choisi parmi Gly, Ser, Ala, Asn, Cys, Gln, His, Ile, Leu, Met, Phe, Pro, Thr, Trp, Tyr, Val et la sélénocystéine ; et flanqué sur l'un ou l'autre côté par au moins un, de préférence au moins deux acides aminés tels que définis en (a).

2. Peptide selon la revendication 1, dans lequel la fraction d'acides aminés qui ont une chaîne latérale qui est chargée positivement à un pH neutre est située dans la plage comprise entre zéro et 0,2, de préférence zéro.

3. Peptide selon la revendication 1 ou 2, lequel peptide est capable de se lier à une nanoparticule magnétique.

4. Peptide selon l'une quelconque des revendications 1 à 3, dans lequel un, plusieurs ou la totalité des acides aminés de la revendication 1(b) sont Gly.

5. Molécule qui est un polypeptide ou une protéine, ledit polypeptide ou protéine étant un une construction de fusion et comprenant au moins une séquence d'un peptide
(i) tel que défini dans l'une quelconque des revendications précédentes ; ou
(ii) consistant en une séquence de 5 à 30, de préférence 6 à 12, tout spécialement 10 à 12 acides aminés, dans laquelle le peptide est capable de se lier à une surface comprenant de l'oxyde de fer magnétique, et dans laquelle
(a) au moins 2/3 desdits acides aminés sont choisis parmi Asp et Glu ; et
(b) au moins un acide aminé qui a une chaîne latérale qui ne porte pas une charge nette à un pH neutre est présent, choisi parmi Gly, Ser, Ala, Asn, Cys, Gln, His, Ile, Leu, Met, Phe, Pro, Thr, Trp, Tyr, Val et la sélénocystéine,
dans laquelle la ou les séquences dans ledit polypeptide ou protéine qui ne comprennent pas ladite au moins une séquence d'un peptide sont hétérologue vis-à-vis de ladite séquence d'un peptide.

6. Molécule liée de manière covalente à au moins une séquence d'un peptide tel que défini dans l'une quelconque des revendications 1 à 5, dans laquelle la liaison covalente n'est pas une liaison peptidique de chaîne principale, laquelle molécule est un polypeptide ou une protéine ou un acide nucléique tel qu'un ADN ou ARN, y compris les ARNsi.

7. Surface comprenant de l'oxyde de fer, de préférence une nanoparticule magnétique, qui est lié à au moins un peptide ou molécule tel que défini dans l'une quelconque des revendications précédentes.

8. Méthode de séparation d'une molécule telle que définie dans la revendication 5 ou 6 d'avec un échantillon, ladite méthode comprenant :
(a) la mise en contact dudit échantillon avec une surface comprenant de l'oxyde de fer, de préférence des nanoparticules magnétiques, dans des conditions permettant la liaison de ladite molécule à ladite surface ; et
(b) la séparation de ladite surface d'avec ledit échantillon ;
en conséquence de quoi ladite molécule est séparée dudit échantillon.

9. Méthode de séparation d'un analyte d'avec un échantillon, ledit analyte étant capable de se lier à une molécule telle que définie dans la revendication 5 ou 6, ladite méthode comprenant :
(a) la mise en contact dudit échantillon, de ladite molécule et d'une surface comprenant de l'oxyde de fer, de préférence de nanoparticules magnétiques, dans des conditions permettant (i) la liaison de ladite molécule à ladite surface et (ii) la liaison dudit analyte à ladite molécule ; et
(b) la séparation de ladite surface d'avec ledit échantillon ;
en conséquence de quoi ledit analyte est séparé dudit échantillon.

10. Méthode selon la revendication 8 ou 9, comprenant en outre
(c) le lavage de ladite surface ; et/ou
(d)
(i) la dissociation de ladite molécule d'avec ladite surface ;
(ii) éventuellement suivie de l'élimination de ladite surface d'avec le résultat de (i).

11. Méthode selon la revendication 10, dans laquelle ladite dissociation dans l'étape (d)(i) est effectuée par addition
(a) d'un acide carboxylique, de préférence d'un acide carboxylique comprenant deux ou trois groupes carboxyliques, tout spécialement d'acide citrique, ledit acide carboxylique étant de préférence en solution tamponnée ; et/ou
(b) d'un oxoacide inorganique, de préférence d'acide phosphorique, ledit oxoacide étant de préférence en solution tamponnée.

12. Méthode de détermination de la présence ou de l'absence d'un analyte dans un échantillon suspecté de comprendre ledit analyte, ledit analyte étant capable de se lier à une molécule telle que définie dans la revendication 5 ou 6, ladite méthode comprenant
les étapes (a) et (b) telles que définies dans la revendication 9,
éventuellement une, plusieurs ou la totalité des étapes (c), (d)(i), (d)(ii) et (e) telles que définies dans les revendications 10 et 11,
ce qui donne ainsi un mélange, et
(f) l'analyse dudit mélange pour la présence dudit analyte.

13. Trousse comprenant ou consistant en
(a)
(i) un peptide tel que défini dans l'une quelconque des revendications 1 à 4 ;
(ii) une molécule telle que définie dans la revendication 5 ou 6 ; et/ou
(iii) un acide nucléique codant un peptide de (i) ou une molécule de (ii), dans lequel ladite molécule est un polypeptide ou une protéine, lequel acide nucléique est de préférence compris dans un vecteur d'expression.

14. Trousse selon la revendication 13, comprenant en outre un, plusieurs ou la totalité des suivants :
(b) une surface comprenant de l'oxyde de fer, de préférence des nanoparticules magnétiques ;
(c) une première solution ou des constituants pour préparer ladite première solution, ladite première solution étant capable d'établir des conditions qui permettent la liaison dudit peptide et/ou de ladite molécule à ladite surface ;
(d) une deuxième solution ou des constituants pour préparer ladite deuxième solution, ladite deuxième solution étant capable d'établir des conditions qui permettent la dissociation dudit peptide et/ou de ladite molécule d'avec ladite surface ; et
(e) des instructions pour l'utilisation de ladite trousse, de préférence pour la mise en oeuvre d'une méthode telle que définie dans l'une quelconque des revendications 8 à 12.
